# EUROPÄISCHE PATENTSCHRIFT

(11) **EP 0 407 961 B1**
(45) Veröffentlichungstag und Bekanntmachung des Hinweises auf die Patenterteilung: **31.03.1993**
(21) Anmeldenummer: 90113146.6
(22) Anmeldetag: 10.07.1990
(51) Int. Cl.: C07C 19/08, B01J 23/26, C07C 17/00, C07C 17/20

(54) **Verfahren zur Herstellung von 1,1,1-Trifluor-2-chlorethan**
Method for the production of 1,1,1-trifluoro-2-chloroethane
Procédé pour la fabrication de 1,1,1-trifluoro-2-chloroéthane

(30) Priorität: 14.07.1989 DE 3923256
(43) Veröffentlichungstag der Anmeldung: 16.01.1991
(73) Patentinhaber: HOECHST AKTIENGESELLSCHAFT, 65926 Frankfurt am Main (DE)
(72) Erfinder: Wanzke, Wolfgang, Dr., D-6230 Frankfurt am Main (DE); Siegemund, Günter, Dr., D-6238 Hofheim am Taunus (DE); Müller, Thomas, Dr., D-6380 Bad Homburg (DE)

(56) Entgegenhaltungen:
- EP-A- 0 130 532
- GB-A- 1 025 759
- US-A- 2 885 427
- US-A- 3 755 477
- PATENT ABSTRACTS OF JAPAN, Band 7, Nr. 46 (C-153)(1191), 23. Februar 1983; & JP-A-57197232

## Beschreibung

Die Erfindung betrifft ein Verfahren zur Herstellung von 1,1,1-Trifluor-2-chlorethan durch Umsetzung von Trichlorethen mit Fluorwasserstoff in der Gasphase.

1,1,1-Trifluor-2-chlorethan (das im folgenden auch als Trifluorchlorethan bezeichnet wird) kann als vielseitiges Zwischenprodukt für die Herstellung anderer Trifluormethylverbindungen eingesetzt werden. 1.1,1,2-Tetrafluorethan ( R 134a), das als chlorfreier Ersatzstoff für Difluordichlormethan (R 12) in der Kälte- und Klimatechnik in Frage kommt, wird beispielsweise durch weiteren Chlor-Fluor-Austausch erhalten. Außerdem lassen sich aus Trifluorchlorethan auch Trifluorethanol, Trifluoracetylchlorid und das Inhalationsanaesthetikum Trifluorchlorbromethan herstellen.

Es ist bereits bekannt, daß Trifluorchlorethan bei der Umsetzung von Trichlorethen mit Fluorwasserstoff an geeigneten Katalysatoren sowohl in der Gasphase als auch in flüssiger Phase erhalten werden kann.

Als Katalysatoren für die Gasphasenreaktion sind vorwiegend Feststoffe beschrieben worden, die entweder ganz aus Chrom(III)verbindungen bestehen oder ein Chrom(III)salz auf einem Trägermaterial wie Aluminiumoxid enthalten.

Die Ergebnisse, die bei den bisher bekannten Herstellverfahren erzielt wurden, sind jedoch in mehrfacher Hinsicht unbefriedigend:
1. Das eingesetzte Trichlorethen wird nur unvollständig umgesetzt.
2. Außer Trifluorchlorethan werden noch verschiedene Nebenprodukte gebildet (mangelnde Selektivität).
3. Die Katalysatoren verlieren rasch an Aktivität.

Die GB-PS 1 025 750 beschreibt die Umsetzung von Trichlorethen mit Fluorwasserstoff an Chromoxifluorid-Katalysatoren, die aus Chrom(III)oxidhydrat bzw. basisch gefälltem Chrom(III)hydroxid hergestellt werden. Mit diesen Katalysatoren wurden die bislang höchsten Umsätze erzielt (86-96 %). Die Ausbeuten an Trifluorchlorethan, bezogen auf umgesetztes Trichlorethen, betrugen 82-92 %.

In der US-PS 3 755 477 wird für einen aus Chrom(III)hydroxid hergestellten Chromoxifluorid-Katalysator, der zusätzlich mit Wasserdampf aktiviert wurde, eine Trifluorchlorethan-Ausbeute von 85 % angegeben.

Untersuchungen mit den beschriebenen Chromoxifluorid-Katalysatoren haben gezeigt, daß nach 50-100 Stunden kontinuierlicher Belastung der Umsatz an Trichlorethen deutlich zurückgeht, so daß diese Katalysatoren für die technische Herstellung von Trifluorchlorethan nicht geeignet sind.

Die in den Patentschriften US-PS 2 885 427 und DE-PS 1 246 703 beschriebenen Verfahren mit anderen chromhaltigen Katalysatoren liefern noch ungünstigere Ausbeuten an Trifluorchlorethan.

Es wurde nun gefunden, daß der in der EP-OS 130 532 = US-PS 4 547 483 beschriebene Katalysator, bestehend aus Magnesiumfluorid und einer fluorhaltigen Chrom(III)-verbindung die Umwandlung von Trichlorethen in Trifluorchlorethan überraschend selektiv und mit nahezu quantitativem Umsatz ermöglicht.

Gegenstand der Erfindung ist ein Verfahren zur Herstellung von 1,1,1-Trifluor-2-chlorethan aus Trichlorethen und Fluorwasserstoff in der Gasphase, dadurch gekennzeichnet, daß ein Chrom und Magnesium enthaltender Katalysator verwendet wird, der dadurch erhältlich ist, daß man Chrom(III)-Hydroxid ausfällt, indem man 1 Mol eines wasserlöslichen Chrom(III)-Salzes mit mindestens 1,5 Mol Magnesiumhydroxid oder Magnesiumoxid in Gegenwart von Wasser umsetzt, die Reaktionsmischung in einen Teig überführt, der Chromhydroxid und ein Magnesiumsalz enthält, und dann den Teig trocknet und bei Temperaturen von 20 bis 500°C mit Fluorwasserstoff behandelt.

Die erfindungsgemäße Gasphasenreaktion ist unter erhöhtem Druck besonders effektiv, da der hohe Umsatz dann über einen besonders langen Zeitraum erhalten bleibt. Während der Reaktion wird im Reaktor durch ein Regelventil ein Druck von 1-26 bar eingestellt, bevorzugt 2-17 bar, besonders bevorzugt 4-10 bar.

Der Katalysator kann bei nachlassender Aktivität durch Überleiten von Luft bei höheren Temperaturen rasch regeneriert werden, so daß er anschließend seine ursprüngliche Leistung wieder erreicht. Dieser Regenerierungsvorgang kann mehrfach wiederholt werden, ohne daß der Katalysator geschädigt wird. Durch eine Vorbehandlung (Formierung) mit Luft bei Temperaturen von 100 bis 500°C wird erreicht, daß der Katalysator zu Beginn der Umsetzung sofort seine maximale Aktivität erreicht.

Das erfindungsgemäße Verfahren kann beispielsweise so durchgeführt werden, daß die Ausgangsstoffe Trichlorethen und Fluorwasserstoff kontinuierlich in einen Verdampfer aus Edelstahl oder Nickel gefördert werden. Die Temperatur des Verdampfers ist nicht kritisch, muß jedoch ausreichen, um beide Komponenten bei dem gewählten Druck vollständig in die Gasphase zu überführen. Die gasförmigen Ausgangsstoffe gelangen, gegebenenfalls über eine Vorheizstrecke und einen Gasmischer, in den Reaktor, der eine Schüttung des in der EP-OS 130 532 beschriebenen Katalysators enthält. Der Reaktor ist ebenfalls aus Edelstahl oder Nickel und kann in verschiedenen technischen Ausführungen eingesetzt werden, z.B. als Schacht-, Röhren- oder Ringspaltreaktor.

Die Temperatur der Katalysatorschüttung wird durch Beheizen des Reaktors bei 150-450°C gehalten, bevorzugt bei 250-350°C.

Um einen möglichst vollständigen Umsatz des Trichlorethens zu erreichen, wird der Fluorwasserstoff bevorzugt im Überschuß eingesetzt. Das Molverhältnis Fluorwasserstoff/Trichlorethen ist i. allg. mindestens 3:1; nach oben ist das Molverhältnis nur durch wirtschaftliche Überlegungen begrenzt. Vorzugsweise liegt es bei 4:1 bis 8:1, insbesondere 4:1 bis 6:1.

Das erfindungsgemäße Verfahren wird durch die nachfolgenden Beispiele näher erläutert.

### Versuchsbericht

### A (Katalysatorherstellung gemäß EP-OS 130 532)

200 g Cr(NO₃)₃ x 9 H₂O wurden in 1 l Wasser gelöst. Diese Lösung wurde zu einer Mischung von 500 g Magnesiumoxid und 240 g Graphit gegeben und die dabei entstehende pastöse Masse innig verknetet.

Anschließend wurde das pastöse Reaktionsprodukt zu Würfelformlingen (0,5 cm Kantenlänge) granuliert und 16 Stunden bei 100°C getrocknet.

1 l (Schüttvolumen) der getrockneten Katalysatorkörper (= 600 g) wurden in einem Rohr aus Nickel oder VA-Stahl mit 5 cm lichter Weite und 100 cm Länge bei 200°C mit 15 mol Fluorwasserstoff behandelt. Die Dauer der Fluorwasserstoffbehandlung betrug ca. 6 Stunden. Dabei wurde das HF mit N₂ verdünnt. Der erhaltene Fluorierungskatalysator wies einen Chromgehalt von 2,3 Gew.-% auf.

### B (Katalysatorherstellung gemäß EP-OS 130 532)

200 g Cr(NO₃)₃ x 9 H₂O wurden in 278 ml Wasser aufgelöst. Diese Lösung wurde zu einer Mischung von 138 g Magnesiumoxid und 136 g Graphit gegeben. Die Weiterverarbeitung und Fluorwasserstoffbehandlung erfolgte gemäß Versuchsbericht A. Der fertige Fluorierungskatalysator enthielt 4,3 Gew.-% Chrom.

### Beispiel 1

56 g Trichlorethen und 43 g Fluorwasserstoff wurden pro Stunde in gasförmigem Zustand über 1 l des nach Versuchsbericht A hergestellten Katalysators in einem Rohrreaktor geleitet, der durch eine elektrische Heizwicklung auf einer Temperatur von 300°C gehalten wurde.

Der Rohrreaktor war der gleiche, der schon zur Fluorwasserstoffbehandlung bei der Herstellung des Katalysators eingesetzt wurde.

Die den Reaktor verlassenden gasförmigen Reaktionsprodukte wurden einer mit Wasser oder Kalilauge gefüllten Waschvorlage zugeführt, in der entstandener Chlorwasserstoff und überschüssiger Fluorwasserstoff aufgefangen wurden.

Die gasförmigen, nicht wasserlöslichen Reaktionsprodukte wurden gaschromatographisch analysiert.

Nach 24 Stunden bei Normaldruck betrug der Umsatz 98,6 %, bezogen auf eingesetztes Trichlorethen. Die Selektivität für Trifluorchlorethan war 98,7 %, bezogen auf umgesetztes Trichlorethen.

Das Reaktionsprodukt enthielt 97,3 Gew.-% CF₃-CH₂Cl.

Nach 170 Stunden bei Normaldruck betrug der Umsatz 95,0 %, die Selektivität lag bei 96,3 %.

### Beispiel 2

Der gemäß Versuchsbericht A hergestellte Katalysator wurde in derselben Versuchsanordnung wie in Beispiel 1 zur Umsetzung von Trichlorethen mit Fluorwasserstoff eingesetzt. Es wurden 120 g pro Stunde Trichlorethen und 92 g pro Stunde Fluorwasserstoff gasförmig bei 300°C über den Katalysator geleitet.

Nach 24 Stunden bei 9 bar Überdruck betrug der Umsatz 99,8 %, bezogen auf eingesetztes Trichlorethen. Die Selektivität für Trifluorchlorethan war 97,7 %, bezogen auf umgesetztes Trichlorethen.

Das Reaktionsprodukt enthielt 97,5 Gew.-% CF₃-CH₂Cl.

Nach 160 Stunden bei 10 bar betrug der Umsatz 99,3 %, die Selektivität lag bei 97,4 %.

### Beispiel 3

Der gemäß Versuchsbericht A hergestellte Katalysator wurde in derselben Versuchsanordnung wie in Beispiel 1 eingesetzt. Es wurden 120 g pro Stunde Trichlorethen und 82 g pro Stunde Fluorwasserstoff gasförmig über den Katalysator geleitet, was einem Molverhältnis HF/C₂HCl₃ von 4,5 : 1 entspricht. Die Reaktionstemperatur betrug 300°C.

Nach 20 Stunden bei 10 bar Druck fand man einen Umsatz von 95,5 %, bezogen auf eingesetztes Trichlorethen, und eine Selektivität für Trifluorchlorethan von 95,6 %, bezogen auf umgesetztes Trichlorethen.

Das Reaktionsprodukt enthielt 91,3 Gew.-% CF₃-CH₂Cl.

### Beispiel 4

1 Liter eines nach Versuchsbericht A hergestellten Katalysators zeigte in derselben Versuchsanordnung wie in Beispiel 1 nach 420 Stunden kontinuierlicher Umsetzung von Trichlorethen mit Fluorwasserstoff bei wechselnden Versuchsbedingungen in einem anschließenden Testversuch (300°C, 10 bar, 120 g/h Trichlorethen, 92 g/h Fluorwasserstoff) folgendes Ergebnis:
- Umsatz:: 91,6 %
- Selektivität:: 93,1 %

Zur Regenerierung des Katalysators wurden bei 300°C pro Stunde 400 l Luft über den Katalysator geleitet (Dauer: 20 Stunden).

Anschließend wurden Trichlorethen und Fluorwasserstoff unter den gleichen Bedingungen wie im Testversuch am Katalysator zur Reaktion gebracht.

Nach 24 Stunden wurde gaschromatographisch folgendes Ergebnis gefunden:
- Umsatz:: 99,8 %
- Selektivität:: 97,7 %

### Beispiel 5

Der gemäß Versuchsbericht B hergestellte Katalysator wurde in derselben Versuchsanordnung wie in Beispiel 1 eingesetzt. Es wurden 56 g pro Stunde Trichlorethen und 43 g pro Stunde Fluorwasserstoff gasförmig bei 300°C über den Katalysator geleitet.

Nach 5 Stunden bei Normaldruck betrug der Umsatz 98,5 %, bezogen auf eingesetztes Trichlorethen. Die Selektivität für Trifluorchlorethan war 97,6 %, bezogen auf umgesetztes Trichlorethen.

Das Reaktionsprodukt enthielt 96,1 Gew.-% CF₃-CH₂Cl

### Beispiel 6

Der gemäß Versuchsbericht A hergestellte Katalysator wurde in derselben Versuchsanordnung wie in Beispiel 1 eingesetzt. Es wurden 105 g pro Stunde Trichlorethen und 80 g pro Stunde Fluorwasserstoff gasförmig über den Katalysator geleitet. Die Reaktionstemperatur betrug 400°C.

Innerhalb von 20 Stunden wurden 1870 g Produkt mit einem CF₃-CH₂Cl-Gehalt von 50,6 Gew.-% erhalten.

### Beispiel 7

Der gemäß Versuchsbericht A hergestellte Katalysator wurde in derselben Versuchsanordnung wie in Beispiel 1 eingesetzt. Es wurden 130 g pro Stunde Trichlorethen und 100 g pro Stunde Fluorwasserstoff gasförmig über den Katalysator geleitet.

Die Reaktionstemperatur betrug 250°C.

Innerhalb von 20 Stunden wurden 2285 g Produkt mit einem CF₃-CH₂Cl-Gehalt von 88,3 Gew.-% erhalten.

## Patentansprüche

1. Verfahren zur Herstellung von 1,1,1-Trifluor-2-chlorethan aus Trichlorethen und Fluorwasserstoff in der Gasphase, dadurch gekennzeichnet, daß ein Chrom und Magnesium enthaltender Katalysator verwendet wird, der dadurch erhältlich ist, daß man Chrom(III)-Hydroxid ausfällt, indem man 1 Mol eines wasserlöslichen Chrom(III)-Salzes mit mindestens 1,5 Mol Magnesiumhydroxid oder Magnesiumoxid in Gegenwart von Wasser umsetzt, die Reaktionsmischung in einen Teig überführt, der Chromhydroxid und ein Magnesiumsalz enthält, und dann den Teig trocknet und bei Temperaturen von 20 bis 500°C mit Fluorwasserstoff behandelt.

2. Verfahren nach Anspruch 1, dadurch gekennzeichnet, daß die Reaktion von Trichlorethen mit Fluorwasserstoff im Temperaturbereich von 150-450°C durchgeführt wird.

3. Verfahren nach Anspruch 1, dadurch gekennzeichnet, daß die Reaktion von Trichlorethen mit Fluorwasserstoff im Temperaturbereich von 250-350°C durchgeführt wird.

4. Verfahren nach einem der Ansprüche 1 bis 3, dadurch gekennzeichnet, daß die Reaktion von Trichlorethen mit Fluorwasserstoff unter einem Druck von 1-25 bar durchgeführt wird.

5. Verfahren nach einem der Ansprüche 1 bis 3, dadurch gekennzeichnet, daß die Reaktion von Trichlorethen mit Fluorwasserstoff unter einem Druck von 2-17 bar durchgeführt wird.

6. Verfahren nach einem der Ansprüche 1 bis 3, dadurch gekennzeichnet, daß die Reaktion von Trichlorethen mit Fluorwasserstoff unter einem Druck von 4-10 bar durchgeführt wird.

7. Verfahren nach einem der Ansprüche 1 bis 6, dadurch gekennzeichnet, daß Fluorwasserstoff und Trichlorethen in einem Molverhältnis von 4 : 1 bis 8 : 1 eingesetzt werden.

8. Verfahren nach einem der Ansprüche 1 bis 7, dadurch gekennzeichnet, daß der Katalysator vor der Umsetzung mit Luft bei Temperaturen von 100-500°C formiert und bei Aktivitätsverlust auf die gleiche Weise regeneriert wird.

## Claims

1. A process for the preparation of 1,1,1-trifluoro-2-chloroethane from trichloroethene and hydrogen fluoride in the gas phase, which comprises using a catalyst containing chromium and magnesium, which catalyst is obtainable by precipitating chromium(III) hydroxide by reacting 1 mole of a water-soluble chromium(III) salt with at least 1.5 mol of magnesium hydroxide or magnesium oxide in the presence of water, converting the reaction mixture into a paste containing chromium hydroxide and a magnesium salt and then drying the paste and treating it at temperatures of 20 to 500°C with hydrogen fluoride.

2. The process as claimed in claim 1, wherein the reaction of trichloroethene with hydrogen fluoride is carried out in the temperature range of 150-450 °C.

3. The process as claimed in claim 1, wherein the reaction of trichloroethene with hydrogen fluoride is carried out in the temperature range of 250-350 °C.

4. The process as claimed in one of claims 1 to 3, wherein the reaction of trichloroethene with hydrogen fluoride is carried out under a pressure of 1-25 bar.

5. The process as claimed in one of claims 1 to 3, wherein the reaction of trichloroethene with hydrogen fluoride is carried out under a pressure of 2-17 bar.

6. The process as claimed in one of claims 1 to 3, wherein the reaction of trichloroethene with hydrogen fluoride is carried out under a pressure of 4-10 bar.

7. The process as claimed in one of claims 1 to 6, wherein hydrogen fluoride and trichloroethene are used in a molar ratio of 4:1 to 8:1.

8. The process as claimed in one of claims 1 to 7, wherein the catalyst is conditioned before the reaction at temperatures of 100-500°C with air and is regenerated in the same manner upon losing activity.

## Revendications

1. Procédé de préparation du 1,1,1-trifluoro-2-chloréthane à partir du trichloréthène et de fluorure d'hydrogène en phase gazeuse, caractérisé en ce qu'on utilise un catalyseur contenant du chrome et du magnésium qui peut être obtenu en précipitant de l'hydroxyde de chrome (III), en faisant réagir 1 mole d'un sel de chrome (III) soluble dans l'eau avec au moins 1,5 mole d'hydroxyde de magnésium ou d'oxyde de magnésium en présence d'eau, en ce qu'on transforme le mélange réactionnel en une pâte qui contient de l'hydroxyde de chrome et un sel de magnésium, puis en ce qu'on sèche la pâte et la traite à une température de 20 à 500°C par fluorure d'hydrogène.

2. Procédé selon la revendication 1, caractérisé en ce que la réaction du trichloréthène avec fluorure d'hydrogène est effectuée dans le domaine de température de 150 à 450°C.

3. Procédé selon la revendication 1, caractérisé en ce que la réaction du trichloréthène avec fluorure d'hydrogène est effectuée dans le domaine de températures de 250 à 350°C.

4. Procédé selon l'une des revendications 1 à 3, caractérisé en ce que la réaction du trichloréthène avec flourure d'hydrogène est effectuée sous une pression de 1 à 25 bars.

5. Procédé selon l'une des revendications 1 à 3, caractérisé en ce que la réaction du trichloréthène avec fluorure d'hydrogène est effectuée sous une pression de 2 à 17 bars.

6. Procédé selon l'une des revendications 1 à 3, caractérisé en ce que la réaction du trichloréthène avec fluorure d'hydrogène est effectuée sous une pression de 4 à 10 bars.

7. Procédé selon l'une des revendications 1 à 6, caractérisé en ce que fluorure d'hydrogène et le trichloréthène sont utilisés sous un rapport molaire de 4:1 à 8:1.

8. Procédé selon l'une des revendications 1 à 7, caractérisé en ce que le catalyseur est activé avant la réaction avec de l'air à une température de 100 à 500°C et est régénéré de la même manière lorsqu'il perd son activité.
